# EUROPEAN PATENT APPLICATION

(11) **EP 4 066 852 A1**
(43) Date of publication of application: **05.10.2022**
(21) Application number: 20892141.1
(22) Date of filing: 26.11.2020
(51) Int. Cl.: A61K 39/00, A61K 39/39, A61K 39/395, A61K 45/00, A61K 45/06, A61K 48/00, A61P 35/00, A61P 35/02, A61P 37/04, A61P 43/00, C07K 7/06, A61K 38/17, A61K 31/713

(54) **PHARMACEUTICAL COMPOSITION**

(30) Priority: 27.11.2019 JP 2019214479
(71) Applicant: Cytlimic Inc., Tokyo, 101-0045 (JP)
(72) Inventor: DOI Shun, Tokyo 101-0045 (JP); SEGAWA Yoshihide, Tokyo 101-0045 (JP); TAMADA Koji, Ube-shi, Yamaguchi 755-8505 (JP); NAGANO Hiroaki, Ube-shi, Yamaguchi 755-8505 (JP); HAZAMA Shoichi, Ube-shi, Yamaguchi 755-8505 (JP)
(74) Representative: Hoffmann Eitle
(86) International application number: PCT/JP2020/043938
(87) International publication number: WO 2021/106978

(57) **Abstract**

The present invention provides a novel technology useful for a cancer vaccine therapy, that is, a pharmaceutical composition wherein a Toll-like receptor agonist, LAG-3 protein, a variant thereof or a derivative thereof, at least one immunogenic agent, and an immune checkpoint inhibitor are administered in combination.

## Description

### Technical Field

The present invention relates to a pharmaceutical composition, and more specifically, a pharmaceutical composition wherein an immunogenic agent is administered in combination with a Toll-like receptor agonist, LAG-3 protein and an immune checkpoint inhibitor.

### Background Art

"Cancer vaccine therapy" is a method for reducing the size of a cancer tumor by administering a cancer antigen directly to a patient to thereby induce an immune response specific to cancer cells within the patient's body. Examples of the cancer antigen to be administered to a patient include a cancer antigen protein itself, a cancer antigen-derived peptide, a nucleic acid encoding either one of them, dendritic cells presenting a cancer antigen, and cancer cells themselves.

To enhance induction of an immune response by a cancer antigen, a cancer antigen is administered together with an adjuvant.

As the adjuvant, e.g., cytokines stimulating various immunocompetent cells and a Toll-like receptor (TLR) agonist are used.

Also, lymphocyte-activation gene 3 (LAG-3) is used as a vaccine adjuvant.

Patent Literature 1 discloses a medicament containing a cancer antigen-derived peptide in combination with a TLR3 agonist, i.e., Poly I:C (polyinosinic-polycytidylic acid) and an LAG-3 protein/IgG fusion protein. TLR3 is one of the molecules of the innate immune system recognizing virus-derived double-stranded RNA to promote production of interferon type I having a strong antiviral action. Poly I:C is a double-stranded RNA analog known as a TLR3 agonist. It is also known that LAG-3 binds to an MHC class II molecule to negatively regulate proliferation of activated T cells and maintenance of T cell homeostasis, thereby playing an important role in regulatory T cells (Treg) function; and is also involved in controlling homeostasis of plasmacytoid dendric cells.

The immune checkpoint inhibitor is a pharmaceutical agent that inhibits the immunosuppressive system to thereby activate anti-tumor immunity (Non Patent Literatures 1 to 3). Examples of the immune checkpoint inhibitors commonly known include anti-PD-1 antibodies such as nivolumab (Patent Literature 2) and pembrolizumab (Patent Literature 3); anti-PD-Ll antibodies such as atezolizumab (Patent Literature 4), durvalumab (Patent Literature 5) and avelumab (Patent Literature 6); and anti-CTLA-4 antibodies such as ipilimumab (Patent Literature 7) and tremelimumab (Patent Literature 8).

### Citation List

### Patent Literatures

Patent Literature 1: International Publication No. WO 2018/070069
Patent Literature 2: International Publication No. WO 2006/121168
Patent Literature 3: International Publication No. WO 2008/156712
Patent Literature 4: International Publication No. WO 2010/077634
Patent Literature 5: International Publication No. WO 2011/066389
Patent Literature 6: International Publication No. WO 2013/079174
Patent Literature 7: International Publication No. WO 2001/014424
Patent Literature 8: International Publication No. WO 2000/037504

### Non Patent Literatures

Non Patent Literature 1: Menon S. et al., Cancers (2016) 8, 106.
Non Patent Literature 2: Pardoll DM., Nat Rev Cancer (2012) 12, 252-264.
Non Patent Literature 3: Wolchok JD., Cell (2015) 162, 937.

### Summary of Invention

### Technical Problem

A main object of the present invention is to provide a novel technology useful for cancer vaccine therapy.

### Solution to Problem

To attain the above object, the present invention provides the following [1] to [84].
[1] A pharmaceutical composition wherein
   a Toll-like receptor agonist,
   LAG-3 protein, a variant thereof or a derivative thereof,
   at least one immunogenic agent, and
   an immune checkpoint inhibitor
   are administered in combination.
[2] The pharmaceutical composition according to [1], wherein the immune checkpoint inhibitor is an anti-PD-1 antibody, an anti-PD-Ll antibody or an anti-CTLA-4 antibody.
[3] The pharmaceutical composition according to [2], wherein the immune checkpoint inhibitor is an anti-PD-1 antibody.
[4] The pharmaceutical composition according to any one of [1] to [3], wherein the Toll-like receptor agonist is a Toll-like receptor 3 agonist or a Toll-like receptor 9 agonist.
[5] The pharmaceutical composition according to [4], wherein the Toll-like receptor agonist is Poly I:C or a salt thereof.
[6] The pharmaceutical composition according to [5], wherein the Poly I:C is Poly ICLC.
[7] The pharmaceutical composition according to any one of [1] to [6], wherein the LAG-3 protein, a variant thereof or a derivative thereof is a fusion protein of LAG-3 protein and IgG.
[8] The pharmaceutical composition according to any one of [1] to [7], wherein the at least one immunogenic agent is at least one cancer antigen-derived peptide.
[9] The pharmaceutical composition according to [8], wherein the at least one cancer antigen-derived peptide is an HSP70-derived peptide or a GPC3-derived peptide.
[10] The pharmaceutical composition according to [9], wherein the HSP70-derived peptide consists of the amino acid sequence represented by SEQ ID NO: 7, and the GPC3-derived peptide consists of the amino acid sequence represented by SEQ ID NO: 16.
[11] The pharmaceutical composition according to any one of [1] to [10], wherein at least one of
   the Toll-like receptor agonist,
   the LAG-3 protein, a variant thereof or a derivative thereof,
   the at least one immunogenic agent, and
   the immune checkpoint inhibitor
   is contained as an active ingredient in a different preparation from a preparation containing at least one of the others, and the preparations are administered at the same time or different times.
[12] The pharmaceutical composition according to any one of [1] to [10], wherein
   the Toll-like receptor agonist,
   the LAG-3 protein, a variant thereof or a derivative thereof,
   the at least one immunogenic agent, and
   the immune checkpoint inhibitor
   are contained as active ingredients in a single preparation and administered.
[13] The pharmaceutical composition according to any one of [1] to [12], for use in a cancer vaccine therapy.
[14] The pharmaceutical composition according to any one of [1] to [12], which is an anticancer drug.
[15] The pharmaceutical composition according to [13] or [14], wherein the cancer is less sensitive or resistant to the immune checkpoint inhibitor.
[16] A pharmaceutical composition wherein
   Poly ICLC,
   a fusion protein of LAG-3 protein and IgG,
   an HSP70-derived peptide consisting of the amino acid sequence represented by SEQ ID NO: 7, and
   an anti-PD-1 antibody
   are administered in combination.
[17] A pharmaceutical composition wherein
   Poly ICLC,
   a fusion protein of LAG-3 protein and IgG,
   an GPC3-derived peptide consisting of the amino acid sequence represented by SEQ ID NO: 16, and
   an anti-PD-1 antibody
   are administered in combination.
[18] A pharmaceutical composition wherein
   a Toll-like receptor agonist,
   LAG-3 protein, a variant thereof or a derivative thereof, and
   an immune checkpoint inhibitor
   are administered in combination.
[19] The pharmaceutical composition according to [18], wherein the immune checkpoint inhibitor is an anti-PD-1 antibody, an anti-PD-Ll antibody or an anti-CTLA-4 antibody.
[20] The pharmaceutical composition according to [19], wherein the immune checkpoint inhibitor is an anti-PD-1 antibody.
[21] The pharmaceutical composition according to any one of [18] to [20], wherein the Toll-like receptor agonist is a Toll-like receptor 3 agonist or a Toll-like receptor 9 agonist.
[22] The pharmaceutical composition according to [21], wherein the Toll-like receptor agonist is Poly I:C or a salt thereof.
[23] The pharmaceutical composition according to [22], wherein the Poly I:C is Poly ICLC.
[24] The pharmaceutical composition according to any one of [18] to [23], wherein the LAG-3 protein, a variant thereof or a derivative thereof is a fusion protein of LAG-3 protein and IgG.
[25] The pharmaceutical composition according to any one of [18] to [24], wherein
   at least one of the Toll-like receptor agonist,
   the LAG-3 protein, a variant thereof or a derivative thereof, and
   the immune checkpoint inhibitor
   is contained as an active ingredient in a different preparation from a preparation containing at least one of the others, and the preparations are administered at the same time or different times.
[26] The pharmaceutical composition according to any one of [18] to [24], wherein
   the Toll-like receptor agonist,
   the LAG-3 protein, a variant thereof or a derivative thereof, and
   the immune checkpoint inhibitor are contained as active ingredients in a single preparation and administered.
[27] The pharmaceutical composition according to any one of [18] to [26], for use in a cancer vaccine therapy.
[28] The pharmaceutical composition according to [27], wherein the cancer is less sensitive or resistant to the immune checkpoint inhibitor.
[29] The pharmaceutical composition according to any one of [18] to [28], to be administered further in combination with at least one immunogenic agent.
[30] The pharmaceutical composition according to [29], wherein the at least one immunogenic agent is at least one cancer antigen-derived peptide.
[31] The pharmaceutical composition according to [30], wherein the at least one cancer antigen-derived peptide is an HSP70-derived peptide or a GPC3-derived peptide.
[32] The pharmaceutical composition according to [31], wherein the HSP70-derived peptide consists of the amino acid sequence represented by SEQ ID NO: 7, and the GPC3-derived peptide consists of the amino acid sequence represented by SEQ ID NO: 16.
[33] The pharmaceutical composition according to any one of [30] to [32], being an anticancer drug.
[34] The pharmaceutical composition according to [33], wherein the cancer is less sensitive or resistant to the immune checkpoint inhibitor.
[35] A pharmaceutical composition wherein
   Poly ICLC
   a fusion protein of LAG-3 protein and IgG, and
   an anti-PD-1 antibody
   are administered in combination.
[36] A treatment method comprising administering, to a subject,
   a Toll-like receptor agonist,
   a LAG-3 protein, a variant thereof or a derivative thereof,
   at least one immunogenic agent, and
   an immune checkpoint inhibitor, in combination.
[37] The treatment method according to [36], wherein the immune checkpoint inhibitor is an anti-PD-1 antibody, an anti-PD-Ll antibody or an anti-CTLA-4 antibody.
[38] The treatment method according to [37], wherein the immune checkpoint inhibitor is an anti-PD-1 antibody.
[39] The treatment method according to any one of [36] to [38], wherein the Toll-like receptor agonist is a Toll-like receptor 3 agonist or a Toll-like receptor 9 agonist.
[40] The treatment method according to [39], wherein the Toll-like receptor agonist is Poly I:C or a salt thereof.
[41] The treatment method according to [40], wherein the Poly I:C is Poly ICLC.
[42] The treatment method according to any one of [36] to [41], wherein the LAG-3 protein, a variant thereof or a derivative thereof is a fusion protein of LAG-3 protein and IgG.
[43] The treatment method according to any one of [36] to [42], wherein the at least one immunogenic agent is at least one cancer antigen-derived peptide.
[44] The treatment method according to [43], wherein the at least one cancer antigen-derived peptide is an HSP70-derived peptide or a GPC3-derived peptide.
[45] The treatment method according to [44], wherein the HSP70-derived peptide consists of the amino acid sequence represented by SEQ ID NO: 7, and the GPC3-derived peptide consists of the amino acid sequence represented by SEQ ID NO: 16.
[46] The treatment method according to any one of [36] to [45], wherein
   at least one of the Toll-like receptor agonist,
   the LAG-3 protein, a variant thereof or a derivative thereof,
   the at least one immunogenic agent, and
   the immune checkpoint inhibitor
   is contained as an active ingredient in a different preparation from a preparation containing at least one of the others, and the preparations are administered at the same time or different times.
[47] The treatment method according to any one of [36] to [45], wherein
   the Toll-like receptor agonist,
   the LAG-3 protein, a variant thereof or a derivative thereof,
   the at least one immunogenic agent, and
   the immune checkpoint inhibitor
   are contained as active ingredients in a single preparation and administered.
[48] The treatment method according to any one of [36] to [47], being a cancer vaccine therapy.
[49] The treatment method according to any one of [36] to [47], for cancer.
[50] The treatment method according to [48] or [49], wherein the cancer is less sensitive or resistant to the immune checkpoint inhibitor.
[51] A treatment method comprising administering, to a subject,
   Poly ICLC
   a fusion protein of LAG-3 protein and IgG,
   an HSP70-derived peptide consisting of the amino acid sequence represented by SEQ ID NO: 7, and
   an anti-PD-1 antibody, in combination.
[52] A treatment method comprising administering, to a subject,
   Poly ICLC
   a fusion protein of LAG-3 protein and IgG,
   a GPC3-derived peptide consisting of the amino acid sequence represented by SEQ ID NO: 16, and
   an anti-PD-1 antibody, in combination.
[53] A method for inducing an immune response specific to cancer cells, comprising administering, to a subject,
   a Toll-like receptor agonist,
   a LAG-3 protein, a variant thereof or a derivative thereof,
   at least one cancer antigen-derived peptide, and
   an immune checkpoint inhibitor, in combination.
[54] The method according to [53], wherein the immune checkpoint inhibitor is an anti-PD-1 antibody, an anti-PD-Ll antibody or an anti-CTLA-4 antibody.
[55] The method according to [54], wherein the immune checkpoint inhibitor is an anti-PD-1 antibody.
[56] The method according to any one of [53] to [55], wherein the Toll-like receptor agonist is a Toll-like receptor 3 agonist or a Toll-like receptor 9 agonist.
[57] The method according to any one of [53] to [56], wherein the Toll-like receptor agonist is Poly I:C or a salt thereof.
[58] The method according to [57], wherein the Poly I:C is Poly ICLC.
[59] The method according to any one of [53] to [58], wherein he LAG-3 protein, a variant thereof or a derivative thereof is a fusion protein of LAG-3 protein and IgG.
[60] The method according to any one of [53] to [59], wherein the at least one cancer antigen-derived peptide is an HSP70-derived peptide or a GPC3-derived peptide.
[61] The method according to [60], wherein the HSP70-derived peptide consists of the amino acid sequence represented by SEQ ID NO: 7, and the GPC3-derived peptide consists of the amino acid sequence represented by SEQ ID NO: 16.
[62] The method according to any one of [53] to [61], wherein at least one of
   the Toll-like receptor agonist,
   the LAG-3 protein, a variant thereof or a derivative thereof,
   the at least one cancer antigen-derived peptide, and
   the immune checkpoint inhibitor
   is contained as an active ingredient in a different preparation from a preparation containing at least one of the others, and the preparations are administered at the same time or different times.
[63] The method according to any one of [53] to [61], wherein
   Toll-like receptor agonist,
   the LAG-3 protein, a variant thereof or a derivative thereof,
   the at least one cancer antigen-derived peptide, and
   the immune checkpoint inhibitor are contained as active ingredients in a single preparation and administered.
[64] The method according to any one of [53] to [63] for treating cancer.
[65] The method according to [64], wherein the cancer is less sensitive or resistant to the immune checkpoint inhibitor.
[66] A method for inducing an immune response specific to cancer cells, comprising administering, to a subject,
   Poly ICLC
   a fusion protein of LAG-3 protein and IgG,
   an HSP70-derived peptide consisting of the amino acid sequence represented by SEQ ID NO: 7, and
   an anti-PD-1 antibody, in combination.
[67] A method for inducing an immune response specific to cancer cells, comprising administering, to a subject,
   Poly ICLC
   a fusion protein of LAG-3 protein and IgG,
   a GPC3-derived peptide consisting of the amino acid sequence represented by SEQ ID NO: 16, and
   an anti-PD-1 antibody, in combination.
[68] Use of
   a Toll-like receptor agonist,
   LAG-3 protein, a variant thereof or a derivative thereof,
   at least one immunogenic agent, or
   an immune checkpoint inhibitor,
   for producing a pharmaceutical composition, wherein
   the Toll-like receptor agonist,
   LAG-3 protein, a variant thereof or a derivative thereof,
   at least one immunogenic agent, and
   and immune checkpoint inhibitor, are administered in combination.
[69] The use according to [68], wherein the immune checkpoint inhibitor is an anti-PD-1 antibody, an anti-PD-L1 antibody or an anti-CTLA-4 antibody.
[70] The use according to [69], wherein the immune checkpoint inhibitor is an anti-PD-1 antibody.
[71] The use according to any one of [68] to [70], wherein the Toll-like receptor agonist is a Toll-like receptor 3 agonist or a Toll-like receptor 9 agonist.
[72] The use according to [71], wherein the Toll-like receptor agonist is Poly I:C or a salt thereof.
[73] The use according to [72], wherein the Poly I:C is Poly ICLC.
[74] The use according to any one of [68] to [73], wherein the LAG-3 protein, a variant thereof or a derivative thereof is a fusion protein of LAG-3 protein and IgG.
[75] The use according to any one of [68] to [74], wherein at least one immunogenic agent is at least one cancer antigen-derived peptide.
[76] The use according to [75], wherein the at least one cancer antigen-derived peptide is an HSP70-derived peptide or a GPC3-derived peptide.
[77] The use according to [76], wherein the HSP70-derived peptide consists of the amino acid sequence represented by SEQ ID NO: 7, and the GPC3-derived peptide consists of the amino acid sequence represented by SEQ ID NO: 16.
[78] The use according to any one of [68] to [77], wherein at least one of
   the Toll-like receptor agonist,
   the LAG-3 protein, a variant thereof or a derivative thereof,
   the at least one immunogenic agent, and
   the immune checkpoint inhibitor
   is contained as an active ingredient in a different preparation from a preparation containing at least one of the others.
[79] The use according to any one of [68] to [77], wherein
   the Toll-like receptor agonist,
   the LAG-3 protein, a variant thereof or a derivative thereof,
   the at least one immunogenic agent, and
   the immune checkpoint inhibitor are contained as active ingredients in a single preparation.
[80] The use according to any one of [68] to [79], wherein the pharmaceutical composition is used for a cancer vaccine therapy.
[81] The use according to any one of [68] to [79], wherein the pharmaceutical composition is an anticancer drug.
[82] The use according to [80], or [81], wherein the cancer is less sensitive or resistant to the immune checkpoint inhibitor.
[83] Use of
   Poly ICLC,
   a fusion protein of LAG-3 protein and IgG,
   an HSP70-derived peptide consisting of the amino acid sequence represented by SEQ ID NO: 16, or
   an anti-PD-1 antibody
   for producing a pharmaceutical composition wherein
   Poly ICLC,
   the fusion protein of LAG-3 protein and IgG,
   an HSP70-derived peptide consisting of the amino acid sequence represented by SEQ ID NO: 7, and
   the anti-PD-1 antibody
   are administered in combination.
[84] Use of
   Poly ICLC,
   a fusion protein of LAG-3 protein and IgG,
   a GPC3-derived peptide consisting of the amino acid sequence represented by SEQ ID NO: 16, or
   an anti-PD-1 antibody
   for producing a pharmaceutical composition wherein
   Poly ICLC,
   the fusion protein of LAG-3 protein and IgG,
   a GPC3-derived peptide consisting of the amino acid sequence represented by SEQ ID NO: 7, and
   the anti-PD-1 antibody
   are administered in combination.

### Advantageous Effects of Invention

The present invention provides a novel technology useful for a cancer vaccine therapy.

### Brief Description of Drawings

[Figure 1] Figure 1 shows a change in tumor volume of mice in individual experimental groups. The vertical axis represents tumor volume (mm³) and the horizontal axis represents days (Day) after transplantation of melanoma cells (Test Example 1). The break in a line observed just before Day 28 means death of mice.
[Figure 2] Figure 2 shows a change in average tumor volume of mice in individual experimental groups (Test Example 1). The vertical axis represents tumor volume (mm³) and the horizontal axis represents days (Day) after transplantation of melanoma cells.
[Figure 3] Figure 3 shows an average tumor volume of mice in individual experimental groups on Day 21 and Day 28 (Test Example 1).
[Figure 4] Figure 4 shows the degree (score) of infiltration by monocytes (lymphocytes) into a tumor site of mice in individual experimental groups on Day 28 (Test Example 1).
[Figure 5] Figure 5 shows a change in the survival rate of mice in individual experimental groups (Test Example 2). The vertical axis represents the survival rate and the horizontal axis represents days (Day) after transplantation of melanoma cells.
[Figure 6] Figure 6 shows a change in the average tumor volume of mice in individual experimental groups (Test Example 3). The vertical axis represents tumor volume (mm³) and the horizontal axis represents days (Day) after transplantation of colorectal cancer cells.
[Figure 7] Figure 7 shows a change in the survival rate of mice in individual experimental groups (Test Example 3). The vertical axis represents survival rates and the horizontal axis represents days (Day) after transplantation of colorectal cancer cells.
[Figure 8] Figure 8 shows a change in the survival rate of mice in individual experimental groups (Test Example 4). The vertical axis represents survival rates and the horizontal axis represents days (Day) after transplantation of colorectal cancer cells.
[Figure 9] Figure 9 shows a change in the survival rate of mice in individual experimental groups (Test Example 5). The vertical axis represents survival rates and the horizontal axis represents days (Day) after transplantation of colorectal cancer cells.

### Description of Embodiments

Now, preferred embodiments for carrying out the present invention will be described with reference to the accompanying drawings. Note that, the embodiments that will be described below are typical embodiments of the present invention, which should not be construed as narrowing the range of the present invention.

A pharmaceutical composition according to the present invention is characterized in that a Toll-like receptor agonist, LAG-3 protein, a variant thereof or a derivative thereof (hereinafter referred to also as "LAG-3 protein or the like"), at least one immunogenic agent and an immune checkpoint inhibitor, are administered in combination.

It is possible that administration of an immunogenic agent in combination with a Toll-like receptor agonist, LAG-3 protein or the like, and an immune checkpoint inhibitor provides a synergistic and excellent immune effect, compared to administration of the Toll-like receptor agonist, LAG-3 protein and immunogenic agent alone, administration of the immune checkpoint inhibitor alone, or single administration of each of these. It is expected that administration of an immunogenic agent in combination with a Toll-like receptor agonist, LAG-3 protein or the like, and an immune checkpoint inhibitor provides a high immune effect even at a low dose at which no effect can be provided by administration of the Toll-like receptor agonist, LAG-3 protein and immunogenic agent alone, administration of the immune checkpoint inhibitor alone, or single administration of each of these.

A pharmaceutical composition according to the present invention is characterized in that a Toll-like receptor agonist, LAG-3 protein, a variant thereof or a derivative thereof (hereinafter referred to also as "LAG-3 protein or the like"), at least one cancer antigen-derived peptide and an immune checkpoint inhibitor are particularly administered in combination.

It is possible that administration of a cancer antigen-derived peptide in combination with a Toll-like receptor agonist, LAG-3 protein or the like, and an immune checkpoint inhibitor provides a synergistic and excellent antitumor effect, compared to administration of the Toll-like receptor agonist, LAG-3 protein and cancer antigen-derived peptide alone, administration of the immune checkpoint inhibitor alone, or single administration of each of these. It is expected that administration of a cancer antigen-derived peptide in combination with a Toll-like receptor agonist, LAG-3 protein or the like, and an immune checkpoint inhibitor provides a high antitumor effect even at a low dose at which no effect can be provided by administration of the Toll-like receptor agonist, LAG-3 protein and cancer antigen-derived peptide alone, administration of the immune checkpoint inhibitor alone, or single administration of each of these.

The present invention also provides a pharmaceutical composition characterized in that a Toll-like receptor agonist and LAG-3 protein or the like are administered in combination with an immune checkpoint inhibitor. The pharmaceutical composition can be administered in combination with at least one immunogenic agent.

It is expected that application of an immune checkpoint inhibitor in combination with a Toll-like receptor agonist and LAG-3 protein to a cancer vaccine therapy with an immunogenic agent provides a high immune effect even in a patient not easily obtaining a therapeutic effect by single administration of the immune checkpoint inhibitor.

It is expected that application of an immune checkpoint inhibitor in combination with a Toll-like receptor agonist and LAG-3 protein to a cancer vaccine therapy particularly with a cancer antigen-derived peptide, induces an immune response specific to cancer cells to provide a high antitumor effect even in a patient not easily obtaining a therapeutic effect by single administration of the immune checkpoint inhibitor.

### [Immune checkpoint inhibitor]

In the present invention, the "immune checkpoint inhibitor" is an medical agent that inhibits the immunosuppressive system and activates tumor immunity.

Examples of the immune checkpoint inhibitor include, but are not particularly limited to, an anti-PD-1 antibody, an anti-PD-Ll antibody and an anti-CTLA-4 antibody. The immune checkpoint inhibitor is preferably an anti-PD-1 antibody and an anti-PD-Ll antibody, and more preferably, an anti-PD-1 antibody.

The "anti-PD-1 antibody" refers to an antibody that specifically binds to PD-1 (programmed cell death-1; CD279; PDCD1) to reduce, inhibit and/or interfere with signal transduction generated by interaction between PD-1 and its binding partner, PD-L1 or PD-L2.

The anti-PD-1 antibody is not particularly limited as long as its clinical efficacy and safety are confirmed. The anti-PD-1 antibody is preferably nivolumab (e.g., International Publication No. WO 2006/121168) and pembrolizumab (e.g., International Publication No. WO 2008/156712).

The "anti-PD-Ll antibody" refers to an antibody that specifically binds to PD-L1 (programmed cell death ligand 1; CD274; B7-H1) to reduce, inhibit and/or interfere with signal transduction generated by interaction between PD-L1 and its binding partner, PD-1 or B7.1 (CD80).

The anti-PD-Ll antibody is not particularly limited as long as its clinical efficacy and safety are confirmed. The anti-PD-Ll antibody is preferably atezolizumab (e.g., International Publication No. WO 2010/077634), durvalumab (e.g., International Publication No. WO 2011/066389) and avelumab (e.g., International Publication No. WO 2013/079174).

The "anti-CTLA-4 antibody" refers to an antibody that specifically binds to CTLA-4 (Cytotoxic T-lymphocyte-associated protein 4; CD152) to reduce, inhibit and/or interfere with signal transduction generated by interaction between CTLA-4 and its binding partner, B7.1(CD80) or B7.2(CD86).

The anti-CTLA-4 antibody is not particularly limited as long as its clinical efficacy and safety are confirmed. The anti-CTLA-4 antibody is preferably ipilimumab (e.g., International Publication No. WO 2001/014424) and tremelimumab (e.g., International Publication No. WO 2000/037504).

### [Toll-like receptor agonist]

In the present invention, the "Toll-like receptor agonist (TLR agonist)" refers to a molecule that binds to any one of Toll-like receptors (TLR) to provide the same stimulus as when a natural ligand binds to TLR.

Examples of the TLR agonist commonly known include those described in OncoImmunology 1: 5, 699-716; August 2012, OncoImmunology 2: 8, e25238; August 2013; Trends in Immunology, Vol. 30, No. 1, 23-32; Immunity 33, October 29, 2010, 492-503; World Journal of Vaccines, 2011, 1, 33-78 and OncoImmunology 1: 6, 894-907, September 2012. Any one of these TLR agonists commonly known in the technical field can be used.

Examples of the TLR agonist include a TLR1 agonist to a TLR10 agonist. Of them, e.g., a TLR3 agonist, a TLR4 agonist, a TLR7 agonist, a TLR8 agonist, a TLR9 agonist or a TLR10 agonist, can be used. The TLR agonist may be in particular a TLR3 agonist or a TLR9 agonist.

The "TLR1 agonist" refers to a molecule that binds to TLR1 to provide the same stimulus as when a natural ligand binds to TLR1. TLR1 recognizes, e.g., a lipoprotein, to activate the innate immune system.

Examples of the TLR1 agonist include lipoproteins, triacylated lipopeptides and zymosan.

The "TLR2 agonist" refers to a molecule that binds to TLR2 to provide the same stimulus as when a natural ligand binds to TLR2. TLR2 recognizes, e.g., acylated lipopeptides to activate the innate immune system.

Examples of the TLR2 agonist include SMP-105, acylated lipopeptides, amphotericin B, atypical LPS, Byglican, Forins, glyco(phospho)lipids, lipoteichoic acid, peptidoglycan, phenol soluble modulin and zymosan.

The "TLR3 agonist" refers to a molecule that binds to TLR3 to provide the same stimulus as when a natural ligand binds to TLR3. TLR3 recognizes virus-derived double-stranded RNA to activate the innate immune system.

Examples of the TLR3 agonist commonly known include, but are not limited to, a synthetic double-stranded polynucleotide, Poly I:C, which is structurally similar to double-stranded RNA. As Poly I:C, a salt thereof may be used; for example, a sodium salt thereof can be used. As Poly I:C, Poly ICLC can be used. As the TLR3 agonist, RIBOXXOL can be used.

Examples of the TLR3 agonist include Poly I:C, Poly ICLC (trade name "Hiltonol"), RIBOXXOL, Ampligen, IPH-3102, cM362-139, and cM362-140.

As the TLR3 agonist, preferably Poly I:C is used. Of Poly I:C, Poly ICLC (trade name, "Hiltonol") stabilized with poly-lysine and carboxymethylcellulose, is more preferably used.

The "TLR4 agonist" refers to a molecule that binds to TLR4 to provide the same stimulus as when a natural ligand binds to TLR4. TLR4 recognizes, a bacterium-derived lipopolysaccharide (LPS) to activate the innate immune system. Examples of the TLR4 agonist commonly known, but are not limited to, MPL. As MPL, a salt thereof may be used; for example, a sodium salt thereof can be used.

Examples of the TLR4 agonist include MPL, MPLA, OM-174 (CRX-527), Picibanil (OK-432) and ONO-4007.

The "TLR5 agonist" refers to a molecule that binds to TLR5 to provide the same stimulus as when a natural ligand binds to TLR5. TLR5 recognizes a bacterium-derived flagellin to activate the innate immune system. Examples of the TLR5 agonist commonly known include, but are not limited to, various bacteria-derived flagellins or a flagellin recombinant protein.

Examples of the TLR5 agonist include CBLB502.

The "TLR6 agonist" refers to a molecule that binds to TLR6 to provide the same stimulus as when a natural ligand binds to TLR6. TLR6 recognizes, e.g., a lipoprotein to activate the innate immune system.

Examples of the TLR6 agonist include diacylated lipopeptides, lipoproteins, lipoteichoic acid and zymosan.

The "TLR7 agonist" refers to a molecule that binds to TLR7 to provide the same stimulus as when a natural ligand binds to TLR7.

The "TLR8 agonist" refers to a molecule that binds to TLR8 to provide the same stimulus as when a natural ligand binds to TLR8.

TLR7 and TLR8 recognize virus-derived single-stranded RNA to activate the innate immune system. Examples of the TLR7/8 agonist commonly known include, but not limited to, imiquimod. As imiquimod, a salt thereof may be used; for example, a sodium salt thereof can be used.

Examples of the TLR7/8 agonist include imiquimod (S-26308, R-837), resiquimod (R-848), 3M-052, 852A, VTX-1463, VTX-2337, AZD8848 (DSP-3025), ANA773 and TMX-101.

The "TLR9 agonist" refers to a molecule that binds to TLR9 to provide the same stimulus as when a natural ligand binds to TLR9. TLR9 recognizes bacterium- and virus-derived CpG DNA to activate the innate immune system. Examples of the TLR9 agonist commonly known include, but are not limited to CpG ODN. As CpG ODN (CpG oligodeoxynucleotide) to be used in the present invention, a salt thereof may be used; for example, a sodium salt thereof can be used.

Examples of the TLR9 agonist include CpG ODN such as CpG, CpG-28, CpG-685 (GNKG168), CpG-1826, CpG-7909 (PF-3512676, agatolimod, Promune (registered trademark)), ODN1585, IMO-2125, IMO-2055 (EMD1201081), ISS1018, MGN-1703, MGN-1706, AVE0675, QAX-935, SAR-21609, SD-101, and DIMS0150.

The "TLR10 agonist" refers to a molecule that binds to TLR10 to provide the same stimulus as when a natural ligand binds to TLR10. TLR10 recognizes, e.g., an acylated lipopeptide, to activate the innate immune system.

Examples of the TLR10 agonist include acylated lipopeptides.

### [LAG-3 protein]

In the present invention, the "LAG-3 protein, a variant thereof or a derivative thereof" refers to LAG-3 protein, a functional variant thereof or functional derivative thereof. LAG-3 protein may be derived from any animal species. However, it is preferable that LAG-3 protein is, for example, a protein derived from the same animal species of a subject to which a pharmaceutical composition according to the present invention is to be administered. Accordingly, if the pharmaceutical composition according to the present invention is administered to a human, huma LAG-3 can be used. LAG-3 protein of a human is a protein having the amino acid sequence represented by NCBI Accession No. P18627.5.

Examples of the functional variant of LAG-3 protein include (i) a LAG-3 variant consisting of an amino acid sequence having an addition, a substitution, or a deletion of one or several amino acids in the amino acid sequence of LAG-3 and having a function of LAG-3 protein required for producing the effect of the present invention, (ii) a LAG-3 variant consisting of an amino acid sequence having a sequence identity of at least 80% or more, or 85% or more, 90% or more, 95% or more, 98% or more, or 99% or more with the amino acid sequence of LAG-3 and having a function of LAG-3 protein required for producing the effect of the present invention, and (iii) a partial polypeptide of LAG-3 protein, a variant (i) above, or a variant (ii) above and having a function of LAG-3 protein required for producing the effect of the present invention.

In the specification, the term "one or several" refers to 1, 2, 3, 4, 5, 6, 7, 8, 9, or 10.

In the present invention, the "amino acid" is used in the broadest sense and includes not only natural amino acids but also artificial amino acid variants and derivatives. Examples of the amino acid include natural proteinous L-amino acids; D-amino acids; chemically modified amino acids such as amino acid variants and derivatives; natural non-proteinous amino acids such as norleucine, β-alanine and ornithine; and chemically synthesized compounds having characteristic features of amino acids known in the technical field. Examples of the non-natural amino acids include α-methylamino acids (e.g., α-methylalanine), D-amino acids, histidine-like amino acids (e.g., β-hydroxy-histidine, homo-histidine, α-fluoromethyl-histidine and α-methyl-histidine), amino acids ("homo" amino acids) having an extra methylene in a side chain and amino acids (e.g., cysteine acid) obtained by substituting a carboxylic acid functional group in a side chain with a sulfonic acid group.

Examples of functional derivatives of LAG-3 protein include a fusion protein of whole or part of LAG-3 protein and other proteins or polypeptide, and LAG-3 protein to which a glycan or a lipid is attached. More specifically, a functional derivative of LAG-3 protein is a fusion protein (LAG-3Ig) of LAG-3 and IgG. Examples of the functional derivative of LAG-3 protein are described in Journal of Translational Medicine 2014, 12:97.

More specifically, the functional derivative of LAG-3 protein preferably used is a fusion protein of LAG-3 and IgG, IMP321 (LAG-3Ig).

### [Immunogenic agent]

In the present invention, the "immunogenic agent" includes a wide variety of substances that can induce an immune response in animal bodies. The immunogenic agent refers to a substance inducing a humoral and/or cellular immune response (having immunogenicity), by administering it to an individual, in such a manner that an antigen-specific immune response against the same or an analogous antigen that (have) encountered the immune system of the individual, increases. An immunogenic agent can induce a cellular immune response such as CD4+T cellular response (for example, TH1, TH2 and/or TH17) and/or CD8+T cellular response (for example, CTL response).

Specific examples of the immunogenic agent include, proteins, glycoproteins, glycans, peptides and nucleic acids (e.g., DNA and RNA). The immunogenic agent may be nucleic acids expressively encoding proteins, glycoproteins or peptides, expression vectors containing a nucleic acid and cells expressing proteins, glycoproteins, glycans or peptides. These immunogenic agents may be derived from disease-causing microbes such as viruses and bacteria, and cancers.

A pharmaceutical composition according to the present invention may contain a single type of immunogenic agent or two or more immunogenic agents.

### [Cancer antigen-derived peptide]

In the present invention, the "cancer antigen-derived peptide" refers to, (i) a peptide having a part of the amino acid sequence of a cancer antigen protein, (ii) a peptide having an amino acid sequence having an addition, a substitution or a deletion of 1 or 2 amino acids in the amino acid sequence (i) and inducing immunity against cancer cells, or (iii) a peptide having a sequence identity of 90% or more, 95% or more, 98% or more, or 99% or more with the amino acid sequence (i) and inducing immunity against cancer cells. A cancer antigen-derived peptide may consist of 8 to 11 amino acid residues. When a cancer antigen-derived peptide is subcutaneously administered to a patient, the peptide is taken up by antigen-presenting cells such as dendritic cells and macrophages, and presented on the cell surfaces together with HLA molecules. Then, cytotoxic T lymphocyte (CTL) progenitor cells, which are reactive to the peptide presented, clonally proliferate. The proliferated/differentiated mature CTL migrate into a cancer tissue through lymph flow. The mature CTL attacks cancer cells expressing a peptide having the same sequence as that of the peptide administered, to induce apoptosis.

A cancer antigen-derived peptide, i.e., HSP70-derived peptide, although it is not particularly limited, is, for example, a peptide described in International Publication No. WO 2018/070069; and more specifically, a peptide containing 8 or more continuous amino acid residues of the amino acid sequence represented by any one of SEQ ID NOs: 1 to 15 and consisting of 11 or less amino acid residues.

As the HSP70-derived peptide, an HSP70-derived peptide having the amino acid sequence particularly represented by any one of SEQ ID NOs: 1 to 15 can be used, and an HSP70-derived peptide having the amino acid sequence represented by SEQ ID NO: 7 is more preferably used.

A cancer antigen-derived peptide, i.e., a GPC3-derived peptide, although it is not particularly limited, is, for example, a peptide described in International Publication No. WO 2018/070069, and more specifically, a peptide containing 8 or more continuous amino acid residues of the amino acid sequence represented by any one of SEQ ID NOs: 16 to 26 and consisting of 11 or less amino acid residues.

As the GPC3-derived peptide, a GPC3-derived peptide having the amino acid sequence particularly represented by any one of SEQ ID NOs: 16 to 26 can be used, and a GPC3-derived peptide having the amino acid sequence represented by SEQ ID NO: 16 is more preferably used.

These antigen-derived peptides are disclosed just as examples. Cancer antigen-derived peptides to be used in a pharmaceutical composition according to the present invention are not limited to these antigen-derived peptides.

A pharmaceutical composition according to the present invention may contain a cancer antigen-derived peptide derived from a single type of cancer antigen protein, and cancer antigen-derived peptides derived from two or more types of cancer antigen proteins. The pharmaceutical composition according to the present invention particularly preferably contains an HSP70-derived peptide having the amino acid sequence represented by SEQ ID NO: 7 and a GPC3-derived peptide having the amino acid sequence represented by SEQ ID NO: 16.

### [Pharmaceutical composition]

A pharmaceutical composition according to the present invention can be applied to a cancer vaccine therapy and can be used as an anticancer drug. The "anticancer drug" herein refers to an agent that can be used for prevention or treatment of a cancer.

A cancer vaccine therapy can be used for prevention or treatment for a cancer. In the present invention, the prevention or treatment for a cancer means resulting in at least one of reducing tumor size, delaying or stopping a tumor from growing, inhibiting (delaying or stopping) cancer metastasis, cancer cells from proliferating and recurrence of cancer, and mitigating one or more symptoms associated with cancer.

The "cancer" is used in the broadest sense. Examples of the cancer include, but are not limited to, astrocytoma, oligodendroglioma, meningioma, neurofibroma, glioblastoma, ependymoma, neurolemmoma, neurofibrosarcoma, neuroblastoma, pituitary tumors (for example, pituitary adenoma), medulloblastoma, melanoma, brain tumors, prostate cancer, head and neck cancer, esophageal cancer, renal cancer, renal cell carcinoma, pancreatic cancer, breast cancer, lung cancer, colon cancer, colorectal cancer, stomach cancer, skin cancer, ovarian cancer, bladder cancer, fibrosarcoma, squamous cell carcinoma, neuroectoderm, thyroid tumor, lymphoma, leukemia, multiple myeloma, hepatocellular carcinoma, mesothelioma and epidermoid carcinoma.

A pharmaceutical composition according to the present invention can be particularly preferably applied to cancer for which existing treatments with an immune checkpoint inhibitor fail to produce a sufficient effect (in other words, cancer low sensitive or resistant to an immune checkpoint inhibitor).

The cancer low sensitive or resistant to an immune checkpoint inhibitor refers to cancer in which infiltration of T lymphocytes into a tumor site rarely occurs and on which an anti-PD-1 antibody (single administration) does not sufficiently produce an antitumor effect. Specific examples of the cancer low sensitive or resistant to an immune checkpoint inhibitor include colorectal cancer, pancreatic cancer, stomach cancer, esophageal cancer, soft tissue sarcoma, ovarian cancer, breast cancer (except triple negative breast cancer), cervical cancer, endometrial cancer, and hepatocellular carcinoma.

A pharmaceutical composition according to the present invention can be applied not only as a systemic therapy to a target but also as a local therapy to a cancer tissue in expectation of a therapeutic effect.

At least one of the components: a Toll-like receptor agonist, LAG-3 protein or the like, an immunogenic agent and an immune checkpoint inhibitor, may be contained as an active ingredient in a different preparation from a preparation containing at least one of the other components, and the preparations are administered at the same time or different times. Alternatively, the individual components may be contained in a single preparation as active ingredients and administered.

Each of the components is dissolved in a water-soluble solvent, formulated in a preparation in the form of a pharmaceutically acceptable salt and can be administered to a patient. Examples of such a pharmaceutically acceptable salt include physiological acceptable water soluble salts such as a sodium salt, a potassium salt, a magnesium salt and a calcium salt. The form of the pharmaceutically acceptable salt may be a buffering solution for keeping physiological pH. Other than the water soluble solvent, a non-water soluble solvent can be used. Examples of the non-water soluble solvent include alcohols such as ethanol and propylene glycol.

A pharmaceutical composition can be orally or parenterally administered. The dosage form of the pharmaceutical composition is not particularly limited. Examples of the dosage form of the pharmaceutical composition include a liquid (for example, injection), a dispersion, a suspension, a tablet, a pill, a powder, a suppository, a powder, a fine grain, a granule, a capsule, a syrup, a nasal drop and an ear drop. Examples of the parenteral administration include intraperitoneal, subcutaneous, intradermal, intramuscular, intravenous and intranasal administrations.

The therapeutically effective amount of a pharmaceutical composition can be appropriately determined depending on the symptom, age, gender, body weight and sensitivity (difference) of the patient, administration method, dosage regimen, the type of preparation and the like.

The therapeutically effective amount per dose is not particularly limited. The therapeutically effective amount per dose of an immune checkpoint inhibitor is, for example, 50 to 800 mg, preferably 100 to 400 mg, and typically 200 mg.

The therapeutically effective amount per dose of a Toll-like receptor agonist is, for example, 0.25 to 5.0 mg, preferably, 0.5 to 2.5 mg, and typically, 1.26 mg.

The therapeutically effective amount per dose of a LAG-3 protein or the like is, for example, 0.25 to 30 mg, preferably, 0.5 to 15 mg, and typically, 1 mg.

The therapeutically effective amount per dose of a cancer antigen-derived peptide per type is, for example, 0.5 to 8 mg, preferably, 1 to 4 mg, and typically, 2 mg.

The therapeutically effective amount per dose of an anti-PD-1 antibody, is, for example, 50 to 800 mg, preferably 100 to 400 mg, and typically, 200 mg.

Administration of an immunogenic agent in combination with a Toll-like receptor agonist, LAG-3 protein or the like, and an immune checkpoint inhibitor possibly reduces individual therapeutically effective amounts of the immunogenic agent, Toll-like receptor agonist, LAG-3 protein or the like, and immune checkpoint inhibitor, compared to administration of the Toll-like receptor agonist, LAG-3 protein and immunogenic agent alone or administration of the immune checkpoint inhibitor alone. In particular, in consideration that an immune checkpoint inhibitor is expensive, expecting (obtaining) the same immune effect at a lower dose is preferable in view of patient's burden and healthcare financing.

Individual components may be all administered at the same time or at least one of the components may be administered at a different time from at least one of the other components. For example, a Toll-like receptor agonist, LAG-3 protein or the like, and a cancer antigen-derived peptide are formulated in a first preparation and an anti-PD-1 antibody in a second preparation, and then, the first and second preparations are administered at different times. In this case, for example, dosage regimen of the first preparation and second preparation can be designed as follows:
First preparation:
   One cycle consists of 28 days (4 weeks).
   In the first cycle and second cycle, the preparation is administered on Day 1, 8, 15 and 22.
   In the third cycle and fourth cycle, the preparation is administered on Day 1 and 15.
   In the fifth cycle and later, the preparation is administered on Day 1.
   Administration is subcutaneously carried out in four sites, i.e., both axillae and both groin areas.
Second preparation:
   One cycle consists of 21 days (3 weeks).
   Administration is intravenously carried out Day 1 of each cycle.

A pharmaceutical composition according to the present invention is formulated by a method commonly known in the technical field.

At this time, at least one of the components: a Toll-like receptor agonist, LAG-3 protein or the like, an immunogenic agent and an immune checkpoint inhibitor may be formulated into a different preparation from a preparation of at least one of the other components. Alternatively, the individual components may be formulated into a single preparation.

In formulating a preparation, pharmacologically acceptable carriers and additives (e.g., excipient, binding agent, dispersant, disintegrant, lubricant, dissolving agent, dissolution aid, colorant, taste and odor masking agent, stabilizer, emulsifier, suspension agent, absorption enhancer, surfactant, pH regulator, preservative and antioxidant) may be used. Examples of the carrier and additives include water, a saline solution, a phosphate buffer solution, dextrose, a pharmacological acceptable organic solvent such as glycerol and ethanol; collagen, polyvinyl alcohol, polyvinylpyrrolidone, carboxy vinyl polymer, sodium carboxymethyl cellulose, sodium polyacrylate, sodium alginate, water-soluble dextran, sodium carboxymethyl starch, pectin, glutamic acid, aspartic acid, methylcellulose, ethyl cellulose, hydroxypropyl cellulose, hydroxypropyl methylcellulose, xanthan gum, gum Arabic, casein, agar, polyethylene glycol, diglycerin, glycerin, propylene glycol, vaseline, paraffin, stearyl alcohol, stearic acid, human serum albumin, mannitol, sorbitol, lactose, glucose, corn starch, microcrystalline cellulose, surfactant, sodium bisulfite, sodium bisulfate, sodium thiosulfate, benzalkonium chloride, chlorobutanol, thimerosal, phenylmercury acetate, phenylmercury nitrate, methylparaben, phenylethyl alcohol, ammonia, dithiothreitol, beta-mercaptoethanol, sodium carbonate, sodium borate, sodium phosphate, sodium acetate, sodium bicarbonate, white sugar, powdered sugar, sucrose, fructose, glucose, lactose, hydrogenated maltose starch syrup, powdered hydrogenated maltose starch syrup, glucose-fructose syrup, fructose-glucose syrup, honey, erythritol, aspartame, saccharin, sodium saccharin and gelatin.

In the case where a pharmaceutical composition according to the present invention contains a peptide, an absorption enhancer for improving transmucosal absorption of the peptide or the like (poorly absorbable drug) can be used. Examples of the absorption enhancer include surfactants such as a polyoxyethylene lauryl ether, sodium lauryl sulfate and saponin; bile salts such as glycolic acid, deoxycholic acid and taurocholic acid; chelating agents such as EDTA and a salicylic acid; fatty acids such as caproic acid, capric acid, lauric acid, oleic acid, linoleic acid and mixed micelle; an enamine derivative, N-acyl collagen peptide, N-acyl amino acid, cyclodextrins, chitosans and a nitric oxide donor.

If a pharmaceutical composition according to the present invention contains a peptide, the composition may be encapsulated in poly(lactic acid)/glycolic acid (PLGA) microcapsules or allowed to adsorb to porous hydroxyapatite micro-particles to formulate into a sustained release preparation; or allowed to transdermally absorb by use of a pulse-release iontophoresis patch system.

If a pharmaceutical composition according to the present invention is applied to a cancer vaccine therapy, an adjuvant may be added. Non-limiting examples of the adjuvant include sedimentary adjuvants such as aluminum hydroxide, sodium hydroxide, aluminum phosphate, calcium phosphate, alum and carboxy vinyl polymer; and oily adjuvants such as Freund's complete adjuvant, Freund's incomplete adjuvant, fluid paraffin, lanolin, Montanide ISA763AV, and Montanide ISA51.

A pharmaceutical composition according to the present invention may be used in combination with an additional anticancer drug, or a radiation therapy or a surgical treatment. Examples of the additional anticancer drug include low molecular weight compounds such as adriamycin, daunomycin, mitomycin, cisplatin, vincristine, epirubicin, methotrexate, 5-fluorouracil, aclacinomycin, nitrogen mustard, cyclophosphamide, bleomycin, daunorubicin, doxorubicin, vincristine, vinblastine, vindesine, tamoxifen, and dexamethasone; and proteins such as cytokine activating immunocompetent cells (for example, human interleukin 2, human granulocyte macrophage colony-stimulating factor, human macrophage colony-stimulating factor, and human interleukin 12).

The additional anticancer drug may be administered simultaneously with, separately or continuously from a pharmaceutical composition according to the present invention, to a subject. The drug and composition may be administered at mutually different time intervals.

### Examples

### [Test Example 1: Antitumor effect on cancer model mice]

The antitumor action of a pharmaceutical composition according to the present invention was examined using cancer model mice.

### [Materials]

Cancer model mice: C57BL/6 mice having mouse melanoma cells (1 × 10⁵ B16F10 cells) subcutaneously transplanted
Poly ICLC: Hiltonol (registered trademark)(Oncovir, Inc.), 50 µg/mouse
LAG-3Ig: IMP321 (Immutep, Ltd.), 1 µg/mouse
GP100 peptide: antigen epitope (SEQ ID NO: 27: EGSRNQDWL) of B16F10 cells, 50 µg/mouse
Anti-PD-1 antibody: InVivoPlus anti m PD-1 (Bio X Cell, Cat. No: BP0146, clone: RPM1-14), 100 µg/mouse

### [Test groups]

The following (1) to (5) experimental groups each consisting of 10 mice were set up.
Control group (1): no treatment
Control group (2): anti-PD-1 antibody alone administered
Control group (3): Poly ICLC, LAG-3Ig and GP100 peptide alone administered
Test group (4): Poly ICLC, LAG-3Ig, GP100 peptide and anti-PD-1 antibody administered
Control group (5): LAG-3Ig, GP100 peptide and anti-PD-1 antibody alone administered

### [Method]

Mouse melanoma cells (1 × 10⁵ B16F10 cells) were subcutaneously transplanted to C57BL/6 mice (Day 0).

Poly ICLC, LAG-3Ig and GP100 peptide were subcutaneously administered to a different site from a tumor transplantation site on Day 0, 5, 12, 19 and 26 (once a week).

The anti-PD-1 antibody was intraperitoneally administered on Day 3, 5, 7, 10, 12 and 14 (three times per week).

A change in tumor volume and infiltration of mononuclear cells (lymphocytes) into a tumor site were evaluated by histopathological methods. The tumor volume was obtained in accordance with the formula:
"(Major axis of tumor tissue) × (Minor axis of tumor tissue)²/2". Lymphocyte infiltration was evaluated based on 3-level scores.

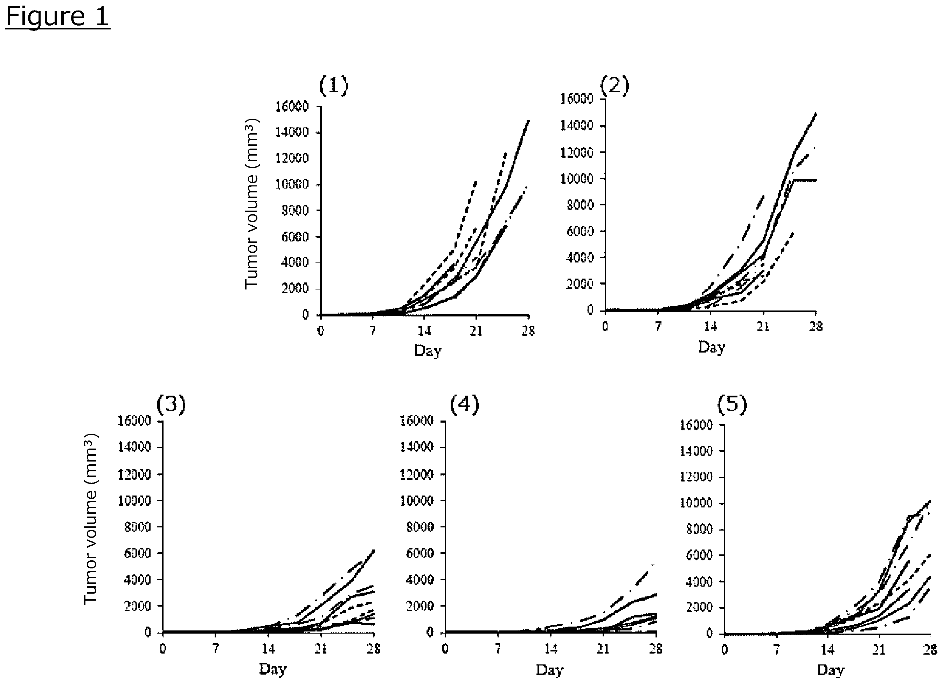

The results are shown in Figures 1-4.

Figure 1 shows a change in tumor volume of mice in individual experimental groups. The vertical axis represents tumor volume (mm³) and the horizontal axis represents days (Day) after transplantation of melanoma cells. The break in a line observed just before Day 28 means death of mice. The number of survival mice up to Day 28 was 3 in control group (1), 3 in control group (2), 9 in control group (3), 9 in test group (4) and 6 in control group (5).

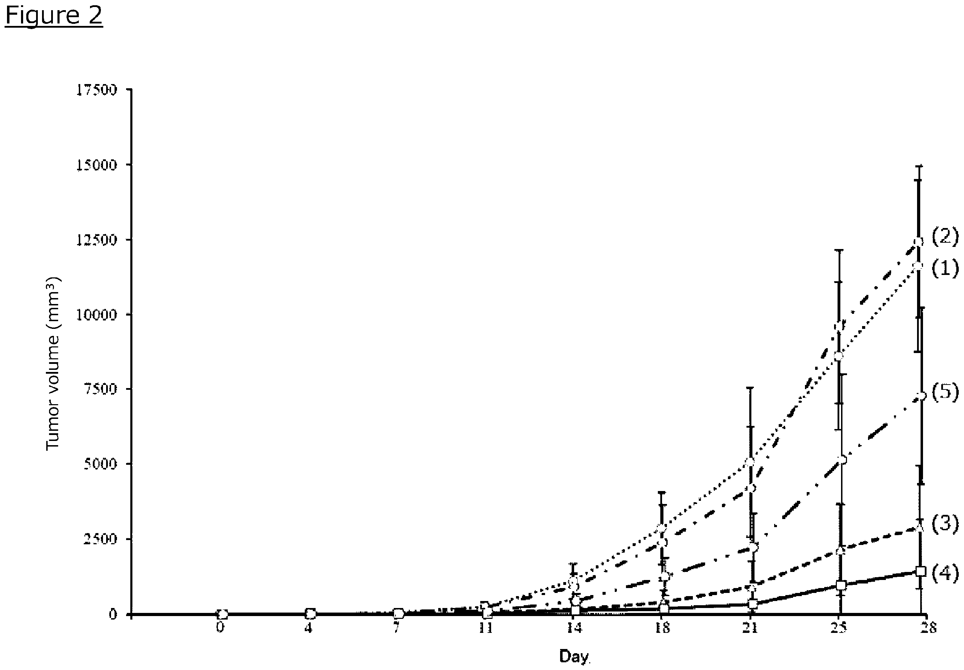

Figure 2 shows a change in average tumor volume of mice in individual experimental groups. The vertical axis represents tumor volume (mm³) and the horizontal axis represents days (Day) after transplantation of melanoma cells.

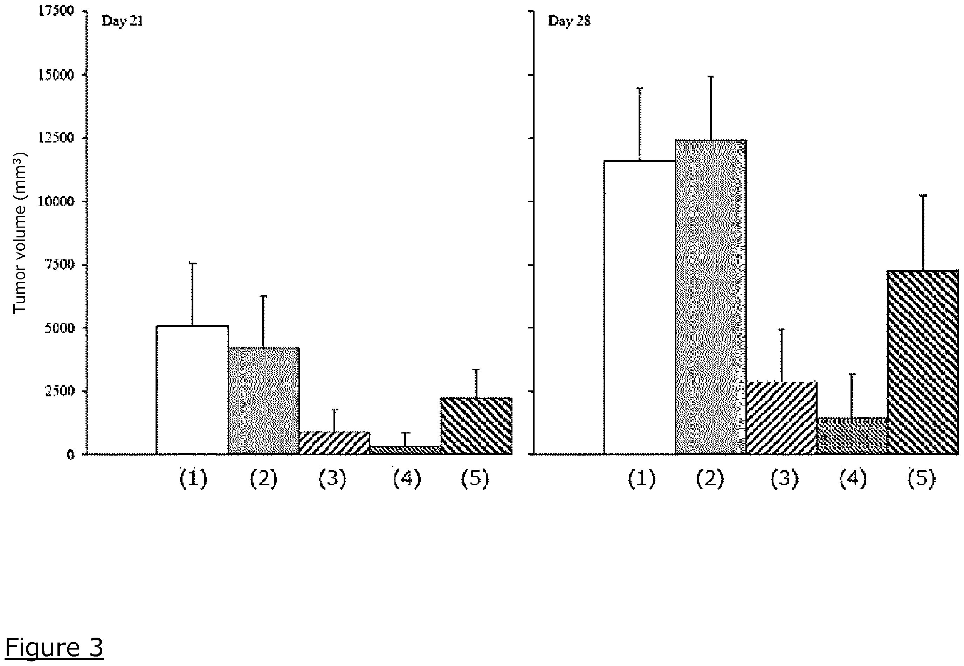

Figure 3 shows an average tumor volume of mice in individual experimental groups on Day 21 and Day 28.

In control group (3) where Poly ICLC, LAG-3Ig and GP100 peptide were administered, a significant antitumor effect was observed, compared to control group (1) where a treatment was not applied and control group (2) where an anti-PD-1 antibody alone was administered.

In control group (2) where an anti-PD-1 antibody alone was administered, proliferation of tumor cells was not virtually suppressed. However, in test group (4) where an anti-PD-1 antibody was administered in addition to Poly ICLC, LAG-3Ig and GP100 peptide, a further significant antitumor effect was observed, compared to control group (3) where Poly ICLC, LAG-3Ig and GP100 peptide alone were administered. The average tumor volume in test group (4) reduced by 64% on Day 21 and by 50% on Day 28 compared to control group (3). It was demonstrated that administration of an anti-PD-1 antibody in combination with Poly ICLC, LAG-3Ig and GP100 peptide produces a synergistic and excellent antitumor effect, compared to administration of Poly ICLC, LAG-3Ig and GP100 peptide alone or administration of an anti-PD-1 antibody alone.

Note that, such an excellent antitumor effect was not obtained in control group (5) where LAG-3Ig, GP100 peptide and anti-PD-1 antibody alone were administered.

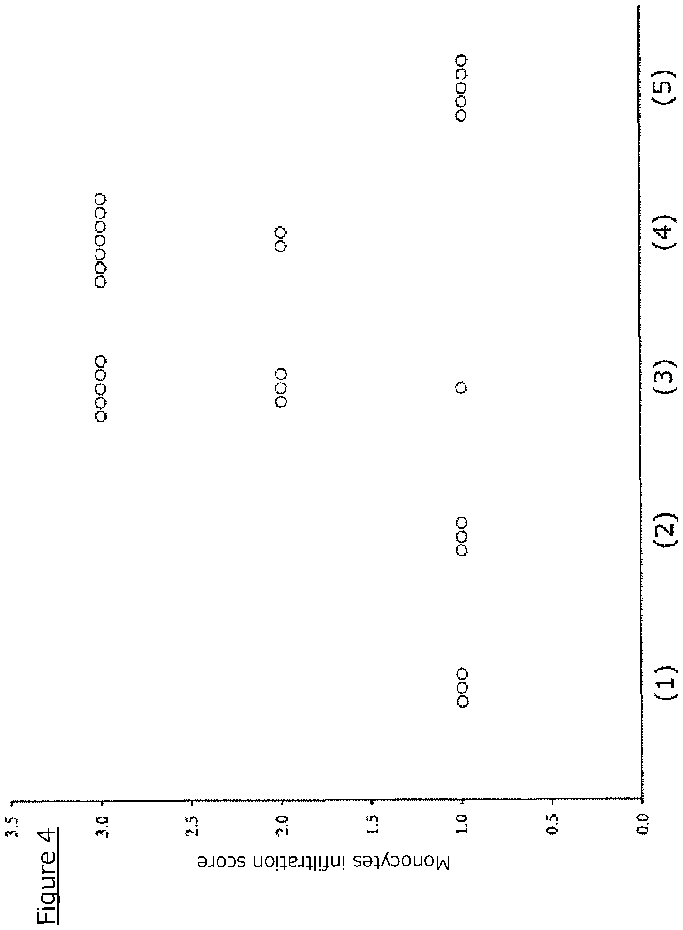

Figure 4 shows the degree (score) of infiltration by monocytes (lymphocytes) into a tumor site of mice in individual experimental groups on Day 28.

In control group (3) where Poly ICLC, LAG-3Ig and GP100 peptide were administered, individuals having a higher degree of infiltration (score 2 or score 3) were observed, compared to control group (1) where a treatment was not applied and control group (2) where an anti-PD-1 antibody alone was administered.

In control group (2) where an anti-PD-1 antibody alone was administered, infiltration was not virtually increased. However, in a test group (4) where an anti-PD-1 antibody was administered in addition to Poly ICLC, LAG-3Ig and GP100 peptide, lymphocyte infiltration was further significant, compared to control group (3) where Poly ICLC, LAG-3Ig and GP100 peptide alone were administered. It was demonstrated that administration of an anti-PD-1 antibody in combination with Poly ICLC, LAG-3Ig and GP100 peptide causes a superior lymphocyte infiltration, compared to administration of Poly ICLC, LAG-3Ig and GP100 peptide alone or administration of an anti-PD-1 antibody alone.

Note that, such a superior lymphocyte infiltration augmentation effect was not observed in control group (5) where LAG-3Ig, GP100 peptide and anti-PD-1 antibody alone were administered.

In the cancer model mice (melanoma B16F10 cells) used in this study, it is known that the degree of infiltration of lymphocytes into a tumor site is low, and single administration of an anti-PD-1 antibody does not provide or does not virtually provide an antitumor effect. It is expected that application of an immune checkpoint inhibitor such as an anti-PD-1 antibody in combination with a Toll-like receptor agonist and LAG-3 protein, to a cancer vaccine therapy with a cancer antigen-derived peptide induces an immune response specific to cancer cells even in a patient not easily obtaining a therapeutic effect by single administration of an immune checkpoint inhibitor to provide a high antitumor effect.

### [Test Example 2: Antitumor effect 2 in cancer model mice]

The test period in Test Example 1 was extended from 28 days to 2 months and the survival rate of cancer model mice was evaluated.

### [Materials]

Cancer model mice: C57BL/6 mice having mouse melanoma cells (1 × 10⁵ B16F10 cells) subcutaneously transplanted
Poly ICLC: Hiltonol (registered trademark) (Oncovir, Inc.), 50 µg/mouse
LAG-3Ig: IMP321 (Immutep, Ltd.), 1 µg/mouse
GP100 peptide: antigen epitope (SEQ ID NO: 27: EGSRNQDWL) of B16F10 cells, 50 µg/mouse
Anti-PD-1 antibody: InVivoPlus anti m PD-1 (Bio X Cell, Cat. No. BP0146, clone: RPM1-14), 100 µg/mouse

### [Test groups]

The following (1) to (5) experimental groups each consisting of 10 mice were set up.
Control group (1): no treatment
Control group (2): anti-PD-1 antibody alone administered
Control group (3): Poly ICLC, LAG-3Ig and GP100 peptide alone administered
Test group (4): Poly ICLC, LAG-3Ig, GP100 peptide and an anti-PD-1 antibody administered
Control group (5): LAG-3Ig, GP100 peptide and an anti-PD-1 antibody alone administered

### [Methods]

Mouse melanoma cells (1 × 10⁵ B16F10 cells) were subcutaneously transplanted to C57BL/6 mice (Day 0).

Poly ICLC, LAG-3Ig and GP100 peptide were subcutaneously administered to a different site from a tumor transplantation site on Day 0, 5, 12, 19 and 26 (once a week).

An anti-PD-1 antibody was intraperitoneally administered on Day 3, 5, 7, 10, 12 and 14 (three times per week).

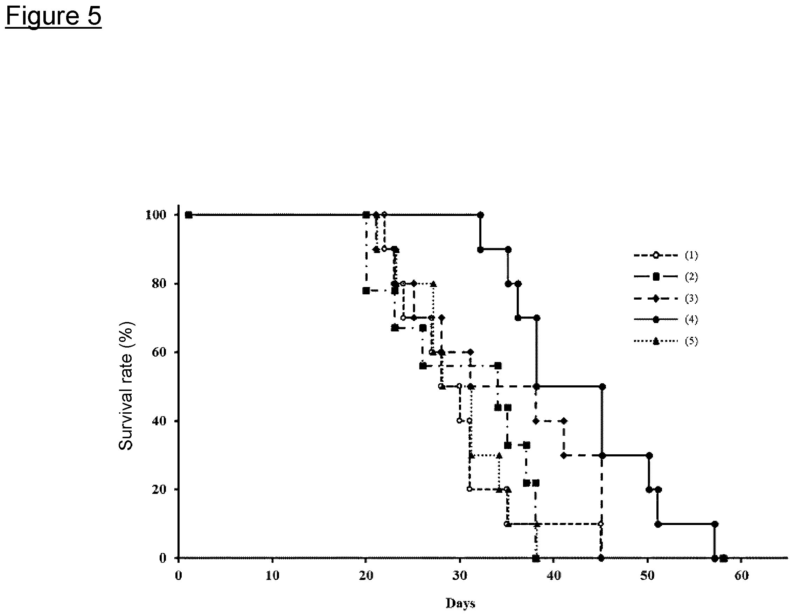

The results are shown in Figure 5.

Figure 5 shows a change in the survival rate of mice in individual experimental groups. The vertical axis represents the survival rate and the horizontal axis represents days (Day) after transplantation of melanoma cells.

In control group (3) where Poly ICLC, LAG-3Ig and GP100 peptide alone were administered, all individuals died up to day 45, similarly to control group (1) where a treatment was not applied. The number of survival days did not increase. In control group (2) where an anti-PD-1 antibody alone was administered, the number of survival days was less than that of control group (1) where a treatment was not applied.

However, in test group (4) where an anti-PD-1 antibody was administered in addition to Poly ICLC, LAG-3Ig and GP100 peptide, the number of survival days significantly increased, compared to control group (2) where an anti-PD-1 antibody alone was administered and control group (3) where Poly ICLC, LAG-3Ig and GP100 peptide alone were administered. The maximum number of survival days of test group (4) was 56. The maximum numbers of survival days of control group (2) and control group (3) were 39 and 46, respectively. It was demonstrated that administration of an anti-PD-1 antibody in combination with Poly ICLC, LAG-3Ig and GP100 peptide produces a synergistic and excellent survival-day extension effect, compared to administration of Poly ICLC, LAG-3Ig and GP100 peptide alone or administration of an anti-PD-1 antibody alone.

Note that, such excellent survival-day extension effect was not obtained in control group (5) where LAG-3Ig, GP100 peptide and anti-PD-1 antibody alone were administered.

### [Test Example 3: Antitumor effect 3 in cancer model mice]

The antitumor action was evaluated by using mouse colon cancer cells (MC38 cells) in place of mouse melanoma cells (B16F10 cells), as the cancer cells to be transplanted to cancer model mice.

### [Materials]

Cancer model mice: C57BL/6 mice having mouse colon cancer cells (1 × 10⁶ MC38 cells) subcutaneously transplanted
Poly ICLC: Hiltonol (registered trademark) (Oncovir, Inc.), 50 µg/mouse
LAG-3Ig: IMP321 (Immutep, Ltd.), 1 µg/mouse
MC38 peptide: a mixture of antigen epitopes of MC38 cells, Reps1 (SEQ ID NO: 28: AQLANDVVL), Adpgk (SEQ ID NO: 29: ASMTNMELM), and Dpagt1 (SEQ ID NO: 30: SIIVFNLL), 50 µg/mouse
Anti-PD-1 antibody: InVivoPlus anti m PD-1 (Bio X Cell, Cat. No. BP0146, clone: RPM1-14), 100 µg/mouse

### [Test groups]

The following (1) to (4) experimental groups each consisting of 5 mice were set up.
Control group (1): no treatment
Control group (2): an anti-PD-1 antibody alone administered
Control group (3): ICLC, LAG-3Ig and MC38 peptide alone administered
Test group (4): Poly ICLC, LAG-3Ig, MC38 peptide and an anti-PD-1 antibody administered

### [Method]

Mouse colon cancer cells (1 × 10⁶ MC38 cells) were subcutaneously transplanted to C57BL/6 mice (Day 0).

Poly ICLC, LAG-3Ig and MC38 peptide were subcutaneously administered to a different site from a tumor transplantation site on Day 5, 12 and 19.

An anti-PD-1 antibody was intraperitoneally administered on Day 5, 12 and 19.

A change in tumor volume was evaluated in the same histopathological method as in Test Example 1.

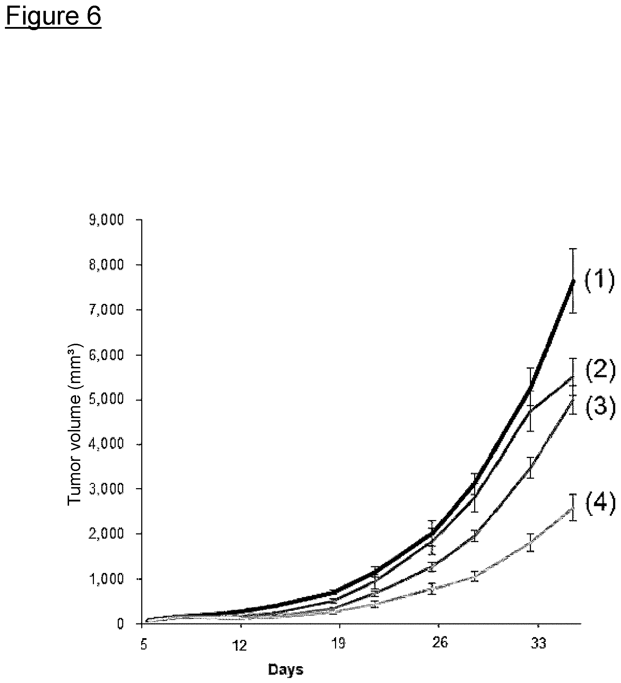

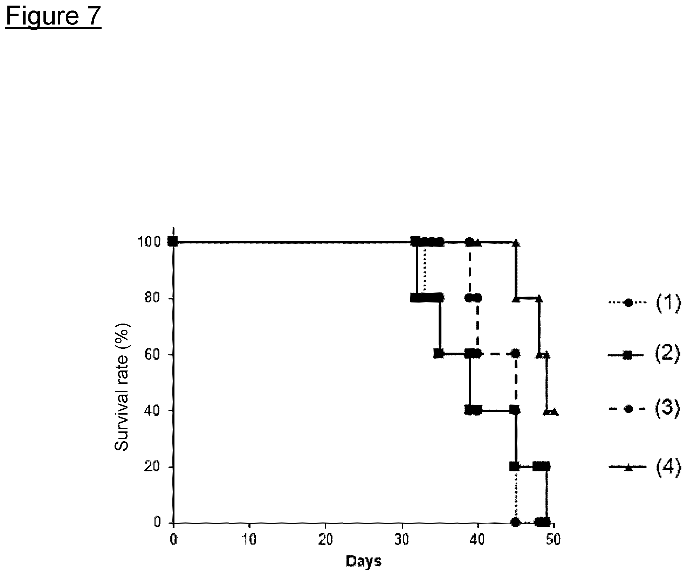

The results are shown in Figures 6 and 7.

Figure 6 shows a change in average tumor volume of mice in individual experimental groups. The vertical axis represents tumor volume (mm³) and the horizontal axis represents days (Day) after transplantation of colorectal cancer cells.

Figure 7 shows a change in the survival rate of mice in individual experimental groups. The vertical axis represents survival rates and the horizontal axis represents days (Day) after transplantation of colorectal cancer cells.

It was confirmed that a tumor size is suppressed in control group (3) where Poly ICLC, LAG-3Ig and MC38 peptide were administered, compared to control group (1) where a treatment was not applied and control group (2) where an anti-PD-1 antibody alone was administered.

In test group (4) where an anti-PD-1 antibody was administered in addition to Poly ICLC, LAG-3Ig and MC38 peptide, a tumor size was further suppressed, compared to control group (3) where Poly ICLC, LAG-3Ig and MC38 peptide alone were administered.

It was demonstrated that administration of an anti-PD-1 antibody in combination with Poly ICLC, LAG-3Ig and MC38 peptide produces synergistic and excellent antitumor effect, compared to administration of Poly ICLC, LAG-3Ig and MC38 peptide alone or administration of an anti-PD-1 antibody alone.

In test group (4) where an anti-PD-1 antibody was administered in addition to Poly ICLC, LAG-3Ig and MC38 peptide, 100% of individuals were alive on Day 45. In contrast, the survival rates of control group (3) where Poly ICLC, LAG-3Ig and MC38 peptide were administered and control group (2) where an anti-PD-1 antibody alone was administered on Day 45 were 60% and 40%, respectively.

Further, in test group (4), 40% of the individuals were alive even on Day 50. In contrast, in control group (3) and control group (2), all individuals died.

It was demonstrated that administration of an anti-PD-1 antibody in combination with Poly ICLC, LAG-3Ig and MC38 peptide produces a survival-day extension effect, compared to administration of Poly ICLC, LAG-3Ig and MC38 peptide alone or administration of the anti-PD-1 antibody alone.

### [Test Example 4: Antitumor effect 4 in cancer model mice]

Antitumor action was evaluated in the same manner as in Test Example 3 except that the dose of the anti-PD-1 antibody was changed from 100 µg/mouse to 66.7 µg/mouse.

Note that, the dose of the anti-PD-1 antibody in humans is determined as about 10 mg/kg body weight degree per time (see, for example, N Engl J Med 2015; 372: 2509-2520). The dose is equivalent to 200 µg/mouse body weight. Accordingly, the dose of 66.7 µg/mouse corresponds to an amount 1/3 of the normal dose of a human.

### [Materials]

Cancer model mice: C57BL/6 mice having mouse colon cancer cells (1 × 10⁶ MC38 cells) subcutaneously transplanted
Poly ICLC: Hiltonol (registered trademark) (Oncovir, Inc.), 50 µg/mouse
LAG-3Ig: IMP321 (Immutep, Ltd.), 1 µg/mouse
MC38 peptide: a mixture of antigen epitopes of MC38 cells, Reps1 (SEQ ID NO: 28: AQLAND WL), Adpgk (SEQ ID NO: 29: ASMTNMELM) and Dpagt1 (SEQ ID NO: 30: SIIVFNLL), 50 µg/mouse
Anti-PD-1 antibody: InVivoPlus anti m PD-1 (Bio X Cell, Cat. No. BP0146, clone: RPM1-14), 66.7 µg/mouse

### [Test group]

The following (1) to (4) experimental groups each consisting of 5 mice were set up.
Control group (1): no treatment
Control group (2): an anti-PD-1 antibody alone administered
Control group (3): Poly ICLC, LAG-3Ig and MC38 peptide alone administered
Test group (4): Poly ICLC, LAG-3Ig, MC38 peptide and anti-PD-1 antibody administered

### [Method]

Mouse colon cancer cells (1 × 10⁶ MC38 cells) were subcutaneously transplanted to C57BL/6 mice (Day 0).

Poly ICLC, LAG-3Ig and MC38 peptide were subcutaneously administered to a different site from a tumor transplantation site on Day 5, 12 and 19.

An anti-PD-1 antibody was intraperitoneally administered on Day 5, 12 and 19.

A change in tumor volume was evaluated in the same histopathological method as in Test Example 1.

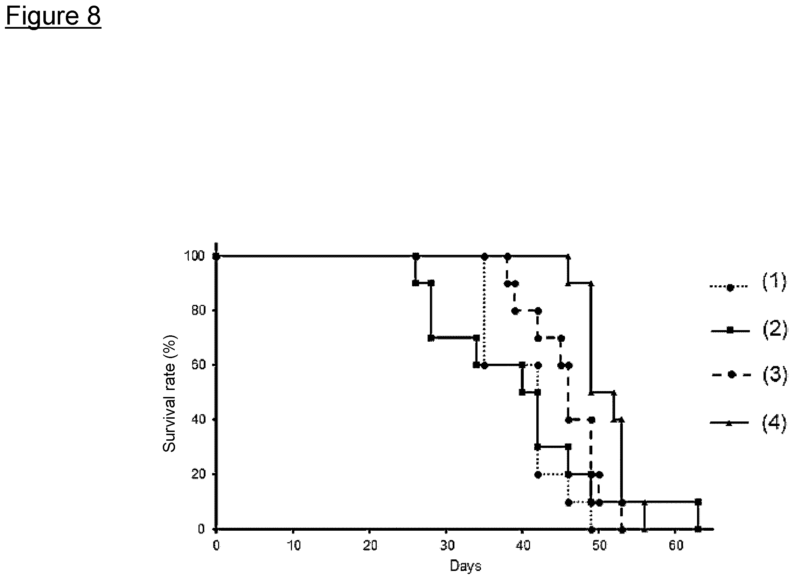

The results are shown in Figure 8.

Figure 8 shows a change in the survival rate of mice in individual experimental groups. The vertical axis represents survival rates and the horizontal axis represents days (Day) after transplantation of colorectal cancer cells.

In test group (4) where an anti-PD-1 antibody was administered in addition to Poly ICLC, LAG-3Ig and MC38 peptide, 100% of individuals were alive on Day 46. In contrast, in control group (3) where Poly ICLC, LAG-3Ig and MC38 peptide were administered and control group (2) where an anti-PD-1 antibody alone was administered, the survival rates on Day 46 were 60% and 30%, respectively.

Further, in test group (4), 40% of the individuals were alive even on Day 53. In contrast, the survival rate of control group (3) and control group (2) on Day 53 were 10% and 10%, respectively.

It was demonstrated that administration of an anti-PD-1 antibody in combination with Poly ICLC, LAG-3Ig and MC38 peptide produces an excellent survival-day extension effect, compared to administration of Poly ICLC, LAG-3Ig and MC38 peptide alone or administration of the anti-PD-1 antibody alone.

### [Test Example 5: Antitumor effect 5 in cancer model mice]

As an experimental group, a group where Poly ICLC was not administered was set up and an antitumor action was evaluated.

### [Materials]

Cancer model mice: C57BL/6 mice having mouse colon cancer cells (1 × 10⁶ MC38 cells) subcutaneously transplanted
Poly ICLC: Hiltonol (registered trademark) (Oncovir, Inc.), 0 or 50 µg/mouse
LAG-3Ig: IMP321 (Immutep, Ltd.), 1 µg or 10 µg/mouse
MC38 peptide: a mixture of antigen epitopes of MC38 cells, Reps1 (SEQ ID NO: 28: AQLANDVVL), Adpgk (SEQ ID NO: 29: ASMTNMELM) and Dpagt1 (SEQ ID NO: 30: SIIVFNLL), 50 µg/mouse
Anti-PD-1 antibody: InVivoPlus anti m PD-1 (Bio X Cell, Cat. No. BP0146, clone: RPM1-14), 66.7 µg/mouse

### [Test groups]

The following (1) to (4) experimental groups each consisting of 5 mice were set up.
Control group (1): no treatment
Test group (4): Poly ICLC, LAG-3Ig, MC38 peptide and an anti-PD-1 antibody were administrated
Control group (5): LAG-3Ig (1 µg), MC38 peptide and an anti-PD-1 antibody alone were administered
Control group (6): LAG-3Ig (10 µg), MC38 peptide and an anti-PD-1 antibody alone were administered

### [Method]

Mouse colon cancer cells (1 × 10⁶ MC38 cells) were subcutaneously transplanted to C57BL/6 mice (Day 0).

Poly ICLC, LAG-3Ig and MC38 peptide were subcutaneously administered to a different site from a tumor transplantation site, on Day 5, 12 and 19.

An anti-PD-1 antibody was intraperitoneally administered on Day 5, 12 and 19.

A change in tumor volume was evaluated in the same histopathological method as in Test Example 1.

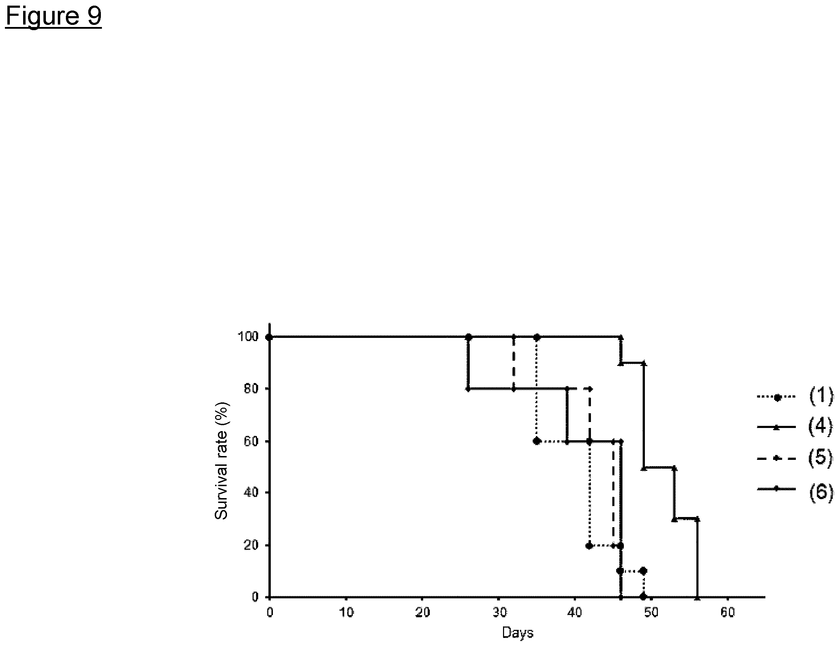

The results are shown in Figure 9.

Figure 9 shows a change in the survival rate of mice in individual experimental groups. The vertical axis represents survival rates and the horizontal axis represents days (Day) after transplantation of colorectal cancer cells.

A survival-day extension effect obtained by an anti-PD-1 antibody in combination with Poly ICLC, LAG-3Ig and MC38 peptide was not obtained by administration of LAG-3Ig, MC38 peptide and an anti-PD-1 antibody alone. The same result was obtained in the case where the dose of LAG-3Ig was increased from 1 µg (control group (5)) to 10 µg (control group (6)) in the same manner as in Test Example 4.

### [Test Example 6: Antitumor effect in cancer patients]

The antitumor action of a pharmaceutical composition according to the present invention is evaluated in accordance with to the following protocol, in phase I/II clinical trials.

### [Name of clinical trial]

Phase I/II clinical trials of an anti-PD-1 antibody in combination with peptide vaccine CYT001 (HSP70-derived peptide, GPC3-derived peptide, Poly ICLC, LAG-3Ig) directed to patients with unresectable and advanced hepatocellular carcinoma

### [Trial design]

Open label and single arm phase I/II trial (safety/efficacy studies) based on Simon's two-stage designs ("Optimal two-stage designs for phase II clinical trials", Simon R, Control Clin. Trials., 1989, 10 (1): 1-10)

### [Target patients]

Subjects are patients (44 cases) with advanced hepatocellular carcinoma satisfying all of the following conditions.
- Histologically or cytologically diagnosed as hepatocellular carcinoma.
- Unresectable
- Incurable or refractory case by standard treatment.
- Allele genotype of HLA-A gene is HLA-A*24: 02, HLA-A*02: 01 or HLA-A*02: 06.
- Physical condition is good (performance status (PS) of the Eastern Cooperative Oncology Group (ECOG) is 0 or 1).
- Liver is in good condition (Child-Pugh score is A).

### [Study drug]

CYT001: 1.7 ml per dose for subcutaneous administration; 1.7 ml of CYT001 contains 2 mg of an HSP70-derived peptide (SEQ ID NO: 16), 2 mg of a GPC3-derived peptide (SEQ ID NO: 7), 1.26 mg of Poly ICLC (Hiltonol (registered trademark)) and 1 mg of LAG-3Ig (IMP321).

Anti-PD-1 antibody: 200 mg per dose for intravascular administration.

### [Dosage regimen]

### CYT001:

One cycle consists of 28 days (4 weeks).

In the first cycle and second cycle, administration is carried out on Day 1, 8, 15 and 22.

In the third cycle and fourth cycle, administration is carried out on Day 1 and 15.

In the fifth cycle and later, administration is carried out on Day 1.

Administration is subcutaneously carried out to four sites: both axillae and both groin areas.

### Anti-PD-1 antibody:

One cycle consists of 21 days (3 weeks).

On the first day in each cycle, intravenous administration is carried out.

It is demonstrated that administration of an anti-PD-1 antibody in combination with Poly ICLC, LAG-3Ig and a cancer antigen-derived peptide produces an excellent antitumor effect. It is expected that application of an anti-PD-1 antibody in combination with a Toll-like receptor agonist and LAG-3 protein to a cancer vaccine therapy with a cancer antigen-derived peptide, induces an immune response specific to cancer cells even in a patient not easily obtaining a therapeutic effect by single administration of an immune checkpoint inhibitor, to provide a high antitumor effect.

### [Sequence listing free text]

SEQ ID NO: 1: Amino acid sequence of an HSP70-derived peptide
SEQ ID NO: 2: Amino acid sequence of an HSP70-derived peptide
SEQ ID NO: 3: Amino acid sequence of an HSP70-derived peptide
SEQ ID NO: 4: Amino acid sequence of an HSP70-derived peptide
SEQ ID NO: 5: Amino acid sequence of an HSP70-derived peptide
SEQ ID NO: 6: Amino acid sequence of an HSP70-derived peptide
SEQ ID NO: 7: Amino acid sequence of an HSP70-derived peptide
SEQ ID NO: 8: Amino acid sequence of an HSP70-derived peptide
SEQ ID NO: 9: Amino acid sequence of an HSP70-derived peptide
SEQ ID NO: 10: Amino acid sequence of an HSP70-derived peptide
SEQ ID NO: 11: Amino acid sequence of an HSP70-derived peptide
SEQ ID NO: 12: Amino acid sequence of an HSP70-derived peptide
SEQ ID NO: 13: Amino acid sequence of an HSP70-derived peptide
SEQ ID NO: 14: Amino acid sequence of an HSP70-derived peptide
SEQ ID NO: 15: Amino acid sequence of an HSP70-derived peptide
SEQ ID NO: 16: Amino acid sequence of a GPC3-derived peptide
SEQ ID NO: 17: Amino acid sequence of a GPC3-derived peptide
SEQ ID NO: 18: Amino acid sequence of a GPC3-derived peptide
SEQ ID NO: 19: Amino acid sequence of a GPC3-derived peptide
SEQ ID NO: 20: Amino acid sequence of a GPC3-derived peptide
SEQ ID NO: 21: Amino acid sequence of a GPC3-derived peptide
SEQ ID NO: 22: Amino acid sequence of a GPC3-derived peptide
SEQ ID NO: 23: Amino acid sequence of a GPC3-derived peptide
SEQ ID NO: 24: Amino acid sequence of a GPC3-derived peptide
SEQ ID NO: 25: Amino acid sequence of a GPC3-derived peptide
SEQ ID NO: 26: Amino acid sequence of a GPC3-derived peptide
SEQ ID NO: 27: Amino acid sequence of GP100 peptide
SEQ ID NO: 28: Amino acid sequence of Reps1 peptide
SEQ ID NO: 29: Amino acid sequence of Adpgk peptide
SEQ ID NO: 30: Amino acid sequence of Dpagt1 peptide

## Claims

1. A pharmaceutical composition wherein
a Toll-like receptor agonist,
LAG-3 protein, a variant thereof or a derivative thereof,
at least one immunogenic agent, and
an immune checkpoint inhibitor
are administered in combination.

2. The pharmaceutical composition according to claim 1, wherein the immune checkpoint inhibitor is an anti-PD-1 antibody, an anti-PD-Ll antibody or an anti-CTLA-4 antibody.

3. The pharmaceutical composition according to claim 1 or 2, wherein the Toll-like receptor agonist is a Toll-like receptor 3 agonist or a Toll-like receptor 9 agonist.

4. The pharmaceutical composition according to claim 3, wherein the Toll-like receptor agonist is Poly I:C or a salt thereof.

5. The pharmaceutical composition according to claim 4, wherein the Poly I:C is Poly ICLC.

6. The pharmaceutical composition according to any one of claims 1 to 5, wherein the LAG-3 protein, a variant thereof or a derivative thereof is a fusion protein of LAG-3 protein and IgG.

7. The pharmaceutical composition according to any one of claims 1 to 6, wherein the at least one immunogenic agent is at least one cancer antigen-derived peptide.

8. The pharmaceutical composition according to claim 7, wherein the at least one cancer antigen-derived peptide is an HSP70-derived peptide or a GPC3-derived peptide.

9. The pharmaceutical composition according to claim 8, wherein the HSP70-derived peptide consists of an amino acid sequence represented by SEQ ID NO: 7, and the GPC3-derived peptide consists of an amino acid sequence represented by SEQ ID NO: 16.

10. The pharmaceutical composition according to any one of claims 1 to 9, wherein at least one of
the Toll-like receptor agonist,
the LAG-3 protein, a variant thereof or a derivative thereof,
the immunogenic agent, and
the immune checkpoint inhibitor
is contained as an active ingredient in a different preparation from a preparation containing at least one of the others, and the preparations are administered at the same time or different times.

11. The pharmaceutical composition according to any one of claims 1 to 9, wherein
the Toll-like receptor agonist,
the LAG-3 protein, a variant thereof or a derivative thereof,
the immunogenic agent, and
the immune checkpoint inhibitor
are contained as active ingredients in a single preparation and administered.

12. The pharmaceutical composition according to any one of claims 1 to 11, for use in a cancer vaccine therapy.

13. The pharmaceutical composition according to any one of claims 1 to 11, which is an anticancer drug.

14. The pharmaceutical composition according to claim 12 or 13, wherein the cancer is less sensitive or resistant to the immune checkpoint inhibitor.

15. A pharmaceutical composition wherein
a Toll-like receptor agonist,
LAG-3 protein, a variant thereof or a derivative thereof, and
an immune checkpoint inhibitor
are administered in combination.

16. The pharmaceutical composition according to claim 14, which is administered further in combination with at least one immunogenic agent.

17. The pharmaceutical composition according to claim 16, wherein the at least one immunogenic agent is at least one cancer antigen-derived peptide.


SEQUENCE LISTING

<110>  CYTLIMIC Inc.

<120>  PHARMACEUTICAL COMPOSITION

<130>  PC53-9005WO

<150>  JP2019-214479
<151>  2019-11-27

<160>  30

<170>  PatentIn version 3.5

<210>  1
<211>  9
<212>  PRT
<213>  Homo sapiens

<400>  1

Gly Val Pro Gln Ile Glu Val Thr Phe
1               5


<210>  2
<211>  9
<212>  PRT
<213>  Homo sapiens

<400>  2

Thr Phe Asp Val Ser Ile Leu Thr Ile
1               5


<210>  3
<211>  9
<212>  PRT
<213>  Homo sapiens

<400>  3

Phe Tyr Pro Glu Glu Ile Ser Ser Met
1               5


<210>  4
<211>  9
<212>  PRT
<213>  Homo sapiens

<400>  4

Lys Leu Leu Gln Asp Phe Phe Asn Gly
1               5


<210>  5
<211>  9
<212>  PRT
<213>  Homo sapiens

<400>  5

Val Leu Val Gly Gly Ser Thr Arg Ile
1               5


<210>  6
<211>  9
<212>  PRT
<213>  Homo sapiens

<400>  6

Met Val Leu Thr Lys Met Lys Glu Ile
1               5


<210>  7
<211>  9
<212>  PRT
<213>  Homo sapiens

<400>  7

Tyr Gly Ala Ala Val Gln Ala Ala Ile
1               5


<210>  8
<211>  9
<212>  PRT
<213>  Homo sapiens

<400>  8

Leu Leu Leu Asp Val Ala Pro Leu Ser
1               5


<210>  9
<211>  9
<212>  PRT
<213>  Homo sapiens

<400>  9

Ala Met Thr Lys Asp Asn Asn Leu Leu
1               5


<210>  10
<211>  9
<212>  PRT
<213>  Homo sapiens

<400>  10

Ile Thr Arg Ala Arg Phe Glu Glu Leu
1               5


<210>  11

<211>  9
<212>  PRT
<213>  Homo sapiens

<400>  11

Asn Leu Leu Gly Arg Phe Glu Leu Ser
1               5


<210>  12
<211>  9
<212>  PRT
<213>  Homo sapiens

<400>  12

Ala Gln Ile His Asp Leu Val Leu Val
1               5


<210>  13
<211>  9
<212>  PRT
<213>  Homo sapiens

<400>  13

Tyr Ala Phe Asn Met Lys Ser Ala Val
1               5


<210>  14
<211>  9
<212>  PRT
<213>  Homo sapiens

<400>  14

Asn Gln Pro Gly Val Leu Ile Gln Val
1               5


<210>  15
<211>  9
<212>  PRT
<213>  Homo sapiens

<400>  15

Ser Val Thr Asn Ala Val Ile Thr Val
1               5


<210>  16
<211>  9
<212>  PRT
<213>  Homo sapiens

<400>  16

Met Val Asn Glu Leu Phe Asp Ser Leu
1               5

<210>  17
<211>  9
<212>  PRT
<213>  Homo sapiens

<400>  17

Leu Phe Asp Ser Leu Phe Pro Val Ile
1               5


<210>  18
<211>  9
<212>  PRT
<213>  Homo sapiens

<400>  18

Ser Ala Leu Asp Ile Asn Glu Cys Leu
1               5


<210>  19
<211>  9
<212>  PRT
<213>  Homo sapiens

<400>  19

Ser Leu Gln Val Thr Arg Ile Phe Leu
1               5


<210>  20
<211>  9
<212>  PRT
<213>  Homo sapiens

<400>  20

Ser Leu Thr Pro Gln Ala Phe Glu Phe
1               5


<210>  21
<211>  9
<212>  PRT
<213>  Homo sapiens

<400>  21

Gly Tyr Ile Cys Ser His Ser Pro Val
1               5


<210>  22
<211>  9
<212>  PRT
<213>  Homo sapiens

<400>  22

Ala Leu Asn Leu Gly Ile Glu Val Ile
1               5

<210>  23
<211>  9
<212>  PRT
<213>  Homo sapiens

<400>  23

Leu Leu Gln Ser Ala Ser Met Glu Leu
1               5

<210>  24
<211>  9
<212>  PRT
<213>  Homo sapiens

<400>  24

Lys Leu Thr Thr Thr Ile Gly Lys Leu
1               5

<210>  25
<211>  9
<212>  PRT
<213>  Homo sapiens

<400>  25

Gly Met Ile Lys Val Lys Asn Gln Leu
1               5

<210>  26
<211>  9
<212>  PRT
<213>  Homo sapiens

<400>  26

Ala Arg Leu Asn Met Glu Gln Leu Leu
1               5

<210>  27
<211>  9
<212>  PRT
<213>  Mus musculus

<400>  27

Glu Gly Ser Arg Asn Gln Asp Trp Leu
1               5

<210>  28
<211>  9
<212>  PRT

<213>  Mus musculus

<400>  28

Ala Gln Leu Ala Asn Asp Val Val Leu
1               5

<210>  29
<211>  9
<212>  PRT
<213>  Mus musculus

<400>  29

Ala Ser Met Thr Asn Met Glu Leu Met
1               5

<210>  30
<211>  8
<212>  PRT
<213>  Mus musculus

<400>  30

Ser Ile Ile Val Phe Asn Leu Leu
1               5